# EUROPEAN PATENT APPLICATION

(11) **EP 2 319 618 A1**
(43) Date of publication of application: **11.05.2011**
(21) Application number: 09761282.4
(22) Date of filing: 10.06.2009
(51) Int. Cl.: B01J 20/26, B01J 20/30, C07D 501/30

(54) **MACROPOROUS ADSORPTION RESIN SPECIAL FOR EXTRACTING CEPHALOSPORIN C AND ITS PREPARATION METHOD**

(30) Priority: 11.06.2008 CN 200810016817
(71) Applicant: Shandong Lukang Record Pharmaceutical Co., Ltd, Jining, Shandong 272000 (CN)
(72) Inventor: WANG, Ling, Shandong 272000 (CN); ZHAO, Xinxiang, Shandong 272000 (CN); SHANG, Xinquan, Shandong 272000 (CN); ZHANG, Yumin, Shandong 272000 (CN); YAN, Xuefeng, Shandong 272000 (CN); QIN, Yuyan, Shandong 272000 (CN)
(74) Representative: Lermer, Christoph
(86) International application number: PCT/CN2009/072212
(87) International publication number: WO 2009/149657

(57) **Abstract**

The invention relates to the macroporous absorbent resin special for extracting cephalosporin C, and process for the preparation is disclosed. Specific surface area of such kinds of adsorbent resins is around 1000-2000m²/g, pore volume is 1.5-2.5ml/g. The preparation method as follows: according to the suspension polymerization to prepare the macroporous copolymer matrix, The mixture of monomer, porogenic agent, initiator of polymerization was put into solution of dissolving some dispersant, then the system was heated to 60-100°C,and maintained for 5-10hours, then the crosslinked macroporous copolymer matrix is obtained. With the existence of inert medium, the above described macroporous copolymer matrix reacted with the Lewis acid as the catalyst at 50-120 °C though the alkylate reaction between the aromatic nucleus. The macroporous absorbent resin special for extracting cephalosporin C descried above possess advantages of high adsorption capacity, good selectivity for target substance, desorption and regeneration easily, and so the high purity of cephalosporin C can be obtained.

## Description

The invention relates to the macroporous absorbent resin special for extracting cephalosporin C, and process for the preparation is disclosed.

### Background of the Invention

Infectious bacterial diseases are common and frequently encountered in our daily life, and up to now kinds of severe bacterial infections are still one potential fatal danger problem. Antibiotics are always getting ranked at the top of variety medicine sold in the hospital all over the world.

The sales condition of the world anti-infective medicine shows that the Cephalosporin comes out on top. Comparing with the penicillin, the Cephalosporin, as the kind of broad spectrum semi-synthesis antibiotics, possesses the virtue of broader antimicrobial spectrum, penicillin-resistant, high effectiveness, low toxicity, less anaphylaxis and so on. The Cephalosporin is an essential factor in the anti-infective therapy. Since the first Cephalosporin product appeared in the market in 1960s, the categories have reached up to more than 60, and the output can cover more than 60% of the yield of antibiotics all over the world. With its high-growing and good clinical manifestation, cephalosporin will cover more and more market share in the field of global anti-infective. Based on the sustainable growth of the domestic economic development at a rate of 15 percent annually, the cephalosporin market is at the stage of rapid development, experts predict that the growing speed will reach up to 30%. The huge market potential attracted lots of investors to set foot in, which expedites the development of whole industry chain from upper stream raw material to pharmaceutical intermediates, etc.

On account of the broad development space, and lots of trendy people are attracted to set foot in, which have brought the development of upstream raw materials and pharmaceutical intermediates, so as to the entire industrial chain can be effectively extended and to be more completed.

Against the background of the prosperous economic, the international agricultural products rising in price results in the cost rising of the pharmaceuticals industry, which promotes the price of the products rising. From July in 2006, the price of the corn sharply rose, in addition with the cost rising of coal and electricity and other power, so many abroad enterprises either stop production or raise the price initiatively. Taking the advantage of labor and environmental cost, China highlights its competitive strength. With the turning development of raw material industry, especially the zymotic raw material, to China, at present, the occident developed countries have stopped the production of cephalosporin C. Our domestic output of the Cephalosporin C has covers 80% of the output all over the world. And now the yearly output of Cephalosporin C from our nine large pharmaceutical domestic corporations comes up to more than 15000 tons.

Cephalosporin C, generally prepared from fermentation, is the key intermediate of the Cephalosporin antibiotics, and as to the extraction of cephalosporin from the fermentation fluid, resin is generally used. The macroporous adsorption resin with the low cost and high productivity, is the best choise for the extraction of Cephalosporin C, and also the operation is simple and convenient, the required facilities are very few, as well it is free from contamination. Above all, due to the tight world power source, and majority of organic solvent price increases, as well as the environment pressure, so the macroporous adsorption resin (commonly know as the polymer sorbent) are used more and more in the pharmaceuticals industry, especially in extracting and refining of antibiotics, extracting of active ingredient from herb and plant, debittering and discoloring of the juice, waste water treatment, etc. At present, the chinese consumption is above 3200 cubic meters, which gross output can reach up to 160,000,000 ¥. The market space is huge. But most of the dedicated resin for extraction of Cephalosporin C which is used by our domestic manufacturer of Cephalosporin C is imported from abroad with high price, and meanwhile the performance of the domestic dedicated resin for Cephalosporin C is just passable, which cannot fully satisfy the requirements of the customer.

U.S. Pat.No.4263407 and 4191813 both state the method of increasing the surface area and porosity of slightly crosslinking gel or macroporous polymer. According to using the organic solvent to swell the slightly crosslinked polymer, macroporous structure is formed with the catalyst of the Lewis acid. This method is quite dependant on the crosslink degree of the original polymer. It requires that the dosage of the cross-linking agent should be controlled between 1-4%, and by adding external cross-linking agent (alkylate agents or acylate agents) or Chloromethylation Reaction with the Lewis acid catalyst to increase the specific surface area and the porosity.

U.S. Pat.No.4543365 states another method about increasing the porosity of the gel or macroporous aromatic crosslinking polymer without chloromethyl, which means the above described spherical copolymer beads will be treated under the condition of the organic solvent with the Lewis acid catalyst, and the required amount of cross-linking agent is around 15-60%. The obtained macroporous crosslinked polymer is used as the dedicated sorbent for Cephalosporin C, and also can be used in preparing of the ion exchange resin or chelate resin after processing of sulphonation or amination through chloromethylation. The U.S. Pat.No.5218004 states the method of treating the high crosslinking styrol copolymer **in water or other unswelling solution**, with taking the Lewis acid as the catalyst and the specific surface area and porosity can be increased according to the reaction of ethylene remaining in the polymer. Both of the patients are utilizing the Lewis acid catalyst to lead to reaction between aromatic nucleus and the suspending ethylene remaining in the matrix, so as to form the new cyclic structure. This new formed pore structure is essential to the increase the specific surface area and porosity.

The above-mentioned different kinds of inventions all emphasized the different methods and mechanisms about postcrosslinking reaction, for increasing the specific surface area and porosity of the crosslinked polymer, so as to produce the crosslinked polymer absorbent with improved properties of adsorption performance, according to the method described above, the specific surface area and porosity have been increased obviously. But if for extracting cephalosporin C, the rein only with good specific surface area and porosity is not enough. At the present time, there's fierce competition of cephalosporin C and downstream product in the current market, meanwhile higher quality of cephalosporin C products are required by users, which results in requiring more severe demand on performance and price of the dedicated resin for extraction of cephalosporin C, so now kinds of the traditional polymer sorbent cannot meet the requirement for the production of cephalosporin C extracted from the fermentation solution which constitutes of impurities with many similar structures and fermentation side-products. So the polymer sorbent is not only requiring with high adsorption capacity, but also requiring with quite good selectivity for cephalosporin C, also it should be with good performance of desorbing and regenerating, which means good pollution resistance, so as to assure the advantages in aspects of quality, yield and cost.

### Summary of the Invention

The present invention provides a preparation method of the dedicated macroporous adsorption resin for extraction of Cephalosporin C. Comparing with the defect of the current technology, this invention possess the higher specific surface area, suitable pore size distribution and porosity, and the unique surface property, so it is quite suitable for extraction of Cephalosporin C.

The design technical scheme as followed:

The structure formula of the Cehpalosporin C is shown as follows in Formula I,

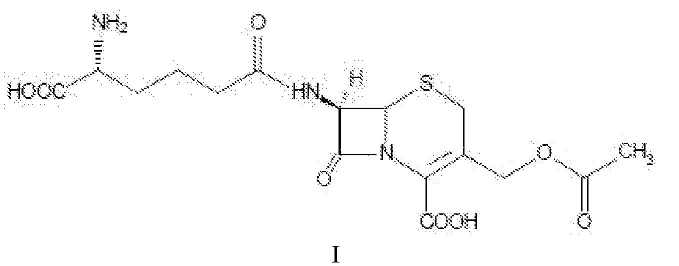

The cephalosporin C exists in the acid solution as the acidic form, which will transform into corresponding salt in the alkalescence condition, according to this property, to adsorb in acidity condition and then desorb in alkalescence solution or compound solution of water and organic solvent, then ferment side-products (such as protein, polypeptide, etc.), pigment and similar impurities can be separated out to realize purification.

The structure of Cephalosporin C shows that the molecular weight is smaller, only 415, and its structure embraces carboxyl, amino and many other polar groups. So the dedicated resin is not only required with high specific surface area and porosity, but also special requirement of structure, size, distribution of pores and its surface polarity.

The dedicated resin with the specific surface area of 1000- 2000m²·g⁻¹, and 1.5-2.5cm³·g⁻¹ of the pore volume, is prepared according to the following method:
(1) According to the suspension polymerization to prepare the macroporous copolymer matrix, The mixture of monomer, porogenic agent, initiator of polymerization was put into solution of dissolving some dispersant, then the system was heated to 60-100 ,and maintained for 5-10hours, then the crosslinked macroporous copolymer matrix is obtained.
(2) With the existence of inert medium, the above described macroporous copolymer matrix reacted with the Lewis acid as the catalyst at 50-120 though the alkylate reaction between the aromatic nucleus.

The following is the detail instruction of the preparation process of appropriative macroporous adsorption resin for extraction of cephalosporin C.

The macroporous adsorption resin used for extraction of cephalosporin C is made according to suspension polymerization to obtain the macroporous copolymer matrix, and then postcrosslinked with the Lewis acid catalyst, as the detail procedure as followed :
1. The mixture of the monomer mixture, porogenic agent and polymerization initiator put into disperse medium solution of quantificationally dissolved dispersant, stired for deconcentration into liquid droplet, and held for 5-10hours to lead to polymerization of monomers at the temperature around 60-100°C, and the liquid droplet will gradually solidify into spherical particle, after the reaction, removal the porogenic agent with azeotropic distillation, after washing and drying, the matrix of crosslinked macroporous copolymer is obtained.
2.With the existence of inert medium, the above described macroporous copolymer matrix reacted with the Lewis acid as the catalyst at 50-120°C for 5-20hours though the alkylate reaction between the aromatic nucleus. Then recycle the inert organic medium to obtain the product. The dosage of inert organic medium is 4-8 times of the quantity of the copolymer matrix , as to the dosage of Lewis acid is about 5-30%.

The following is the description of material constitutes and amount which mentioned in step 1.

The mentioned monomer mixture constitutes of monovinyl aromatic monomer,polyvinyl aromatic monomer used as crosslinking agent and the polar aliphatic unsaturation monomer.

The mentioned monovinyl aromatic monomer includes, for example, styrene, alkyl-substituted styrene, vinylnaphthalene or other similar unsaturated monomer, and as to the alkyl-substituted styrene, there are also several selections as following: methyl styrene, ethylstyrene, diethylstyrene, methylethylstyrene or dimethylstyrene and so on. The most optimized choice of monovinyl aromatic monomer is styrene or alkyl-substituted styrene.

The mentioned polyvinyl aromatic monomer used as the crosslinking agent, such as divinylbenzene, trivinylbenzene, divinyltoluene, divinylnaphthalene, divinylxylene or divinylethylbenzene and so on, the most optimized is divinylbenzene.

The above-mentioned polar aliphatic unsaturated monomer can be selected among the followings: acrylic acid, methacrylic acid, low alkyl ester of acrylic acid or low alkyl ester of methacrylic acid ,and the low alkyl ester of acrylic acid can be selected among methyl acrylate, ethyl acrylate, propyl acrylate, isopropyl acrylate or acrylonitrile; as to the low alkyl ester of methacrylic acid can be selected among the followings: ethyleneglycol di(meth)acrylate, trimethylolpropane tri(meth)acrylate, ethyl methacrylate, propyl methacrylate, isopropyl methacrylate , and the most optimized is low alkyl ester of methacrylic acid or acrylonitrile.

The proportion of polar aliphatic unsaturated monomer in the mixture described above is around 0-10%wt; and the proportion of polyvinyl aromatic monomer is 50-80%wt, more preferably around 60-70%wt; and the others are monovinyl aromatic monomer.

The porogen agent which is used for preparing the ground substance of crosslinked macroporous copolymer can be selected one or more among the followings: toluene, xylene, C₄-C₁₀ alkanol, C₆-C₁₂ saturated hydrocarbon, alkyl-substituted cyclohexane; C₄-C₁₀ alkanol can be selected among n-butanol, tertiary amyl alcohol, or diethyl hexanol ; C₆-C₁₂ saturated hydrocarbon can be selected among heptane, isooctane or gasoline, and other alkyl-substituted cyclohexane.

The optimized porogen can be selected two or three among the followings: toluene, C₄-C₁₀ alkanol, gasoline, or alkyl-substituted cyclohexane.

The amount of the optimized porogen agent is 100-300%wt of the total monomers mixture.

As to the dispersive medium and dispersant please refer to the current technology, and this invention will mention the following optimizing proposal:
The mentioned disperse medium is water or salt water, and then dissolved dispersant of glutin, PVA or carboxymethyl cellulose (CMC), etc.. The initiator of polymerization can be selected among the followings: benzoperoxide, lauroyl peroxide or AZO compound, such as azobisisobutyronitrile, etc., and the amount of initiator is 0.5-2% wt of the total monomers.

As to constituents described in step 2, current technology can be taken as reference, and this invention will mention the following optimizing scheme:
The inert organic medium can be selected among dichloroethane, dichloropropane, nitrobenzene, chlorobenzene or dichloro benzene and so on.

The Lewis acid catalyst can be selected among the followings: anhydrous aluminium trichloride, anhydrous ferric chloride, anhydrous zinc chloride, stannic chloride or boron trifluoride and so on.

The method of recycling the inert organic medium in step 2, is to get the clear solution by siphon method, then add alcohols with low molecular weight or ketones solvent to wash the particles, finally wash with water. Methanol or ethanol is optimized for alcohols with low molecular weight, as to the ketones solvent, acetone is the best choice.

This invention related with macroporous adsorption resin dedicated for extraction of cephalosporin C, which is also named as polymer sorbent, is mainly used in the application of extraction of cephalosporin C.

Based on the postcrosslinking technology , by controlling the degree of crosslinking of polymer matrix, using the porogenic agent, and polar monomers to get the dedicated resin with 1000-2000m²/g specific surface area and 1.5-2.5ml/g pore volume. The macroporous adsorption resin made by this invention method, not only possess the high adsorbance, and comparing with the other resins present used, but also possess better selectivity of cephalosporin C, and higher desorbing ratio, as so the purity and productivity of the target product can be raised. Meanwhile, with the improvement of anti-pollution performance, the prolonged service life will realize its industrial application value better.

Under the extraction features of cephalosporin C, the kinds of dedicated resin for extraction of cephalosporin C with high absorbability and good selectivity of the target product, easily to desorb and regenerate in order to get the high purity cephalosporin C were invented, and because of its high specific surface area, proper pore-size distribution and porosity, and unique surface character, it can be used as the innovative macroporous adsorption resin for extraction of cephalosporin C.

In order that those skilled in the art many more readily understand the present invention and certain preferred embodiments by which it may be carried into effect, the following specific examples are afforded.

### EXAMPLE 1

An aqueous phase of 500ml deionized water and 3.5g gelatin was stirred into solution in a reaction vessel. An organic phase of 75g (80%)divinyl-benzene, 8g methyl methacrylate, 17g styrene, 90g toluene, 50g tertiary amyl alcohol and 1.0g benzoyl peroxide was added to vessel , and the mixture was stired until forming even liquid drops, the temperature was increased to 70-75 ,and maintained for 4 hours, then raise up to 85, standed for 4 hours. The mother liquor was removaled and then the porogenic agent (toluene and tertiary amyl alcohol) was get out by azeotropic distillation until little oily matter left in the condensate, the particles were washed with lots of water after the temperature cooled, then 90g matrix of crosslinked macroporous polymer with 20-60 mesh is obtained after draining water and drying with hot wind till water content is under 1.0%
the above mentioned 90g crosslinked matrix of macroporous copolymer was swollen in 450ml nitrobenzene at the room temperature for 5 hours, then added 14g ferric chloride, the temperature increased to 100-105 , and maintained for 10hours, then cool the temperature, The mother liquor was removaled and the system was washed 2 times with 500ml alcohol under stirring 30minutes one time, then 270g of the brownish-red target product the dedicated macroporous adsorption resin for extraction of cephalosporin C, is obtained and with 1189m²/g specific surface area and 1.78ml/g pore volume after washing by lots of deionized till clear liquid is obtained.

### EXAMPLE 2:

The 95g matrix of crosslinked macroporous copolymer were prepared by the method described in Example 1 but the copolymer beads having 85g (80%) divinylbenzene, 5g methyl acrylate, 15g styrene, 100g toluene, 30g tertiary amyl alcohol, 30g isooctane and 1.0g benzoperoxide.

The postcrosslinked process was also prepared by the method described in Example 1 but the copolymer beads was swollen in 570ml dichloroethane for 2hours at room temperature, and with 19g zinc chloride, the temperature stayed at 80 for 15hours. 300g brownish yellow target product--macroporous absorbent resin special for extracting cephalosporin C, is obtained, and the surface area is 1205m2/g, pore volume is 1.84ml/g.

### Application test

Make the comparing test of adsorption of cephalosporin C between 1# sample resin obtained from embodiment 2, and 2# sample resin of S-825 and 3# sample resin X-18 used in the market.

Test condition: Laboratory column flow-through experiments were performed using glass columns. 100ml Resins were wet-packed to avoid entrapped air bubbles within the column individually. Resins standed after processing the regular pretreatment; the fermentation fluid of cephalosporin adopting the one prepared by organic ultrafiltration membrane was quantificationally injected into the glass column at a constant rate of 1.0 ∼ 1.2BV/hr, then desorbed with weak caustic solution at rate of 0.8 ∼ 1BV/hr. The experimental result presented in Table 1(average value of several batches sample test data), and evaluation for resins in Table 2.

**Table 1: Result of adsorption comparing test for extracting cephalosporin C with 3 resin samples**

| Resin | 1# | 2# | 3# |
|---|---|---|---|
| Injection volume of filtrate ( g/ L resin) | 45.20 | 45.20 | 45.20 |
| Adsorption capacity ( g/L resin ) | 42.67 | 43.10 | 42.40 |
| The total amount of desorbing solution(g/ L resin) | 39.50 | 39.15 | 39.06 |
| Desorption rate(%) | 92.6 | 90.8 | 92.1 |
| Productivity of desorption solution(%) | 87.4 | 86.6 | 86.4 |
| Material balance (%) | 93.0 | 91.3 | 92.4 |
| CPC concentration in desorbing solution (g/L) | 9.47 | 9.51 | 9.39 |
| CPC content in desorbing solution (%) | 90.8 | 89.5 | 90.4 |
| DCPC content in desorbing solution (%) | 3.05 | 3.82 | 3.43 |
| DOCPC content in desorbing solution (%) | 1.72 | 2.00 | 1.96 |
| Ratio of abs of desorbing solution | 3.84 | 3.94 | 4.05 |

| | | | |
|---|---|---|---|
| Note: 1: CPC is cephalosporin C; DCPC and DOCPC are two biggest impurities in the target productor which structures are similar to CPC, individually are deacetylation CPC and deacetoxyl CPC. 2: Ratio of abs of desorbing solution, which mainly reflect clarity and color of desorbing solution, is ratio of absorbance of desorbing solution and concentration of CPC, | | | |

**Table 2: Performance evaluation of 3 sample resins**

| Items | 1# | 2# | 3# |
|---|---|---|---|
| Adsorption capacity | mediate | high | low |
| Output quantity of desorbing solution | high | mediate | lower slightly |
| Productivity of desorbing solution | high | mediate | lower slightly |
| Level of difficulty of elution | easy | difficult | mediate |
| Recovery percent of CPC | high | low | mediate |
| Purity of CPC in desorbing solution | high | worse | mediate |
| DCPC concentration in desorbing solution | low | high | mediate |
| DOCPC concentration in desorbing solution | low | high | mediate |
| Extinction ratio | low | mediate | high |
| Expansion coefficient of menstruum phase | small | mediate | big |
| Comprehensive evaluation | better | worse | mediate |

## Claims

1. The dedicated resin for extracting cephalosporin C, with the specific surface area of 1000-2000m²·g⁻¹, and 1.5-2.5cm³·g⁻¹ of the pore volume, is prepared according to the following method:
( 1 ) According to the suspension polymerization to prepare the macroporous copolymer matrix, The mixture of monomer, porogenic agent, initiator of polymerization was put into solution of dissolving some dispersant, then the system was heated to 60-100°C,and maintained for 5-10hours, then the crosslinked macroporous copolymer matrix is obtained.
( 2 ) With the existence of inert medium, the above described macroporous copolymer matrix reacted with the Lewis acid as the catalyst at 50-120°C though the alkylate reaction between the aromatic nucleus.

2. A method of preparing dedicated resin for extracting cephalosporin C which obtained according to suspension polymerization, then postcrosslinked with the catalysis of Lewis acid is as followed:
(1). The mixture of the monomer mixture, porogenic agent and polymerization initiator put into disperse medium solution of quantificationally dissolved dispersant, stired for deconcentration into liquid droplet, and held for 5-10hours to lead to polymerization of monomers at the temperature around 60-100°C, and the liquid droplet will gradually solidify into spherical particle, after the reaction, removal the porogenic agent with azeotropic distillation, after washing and drying, the matrix of crosslinked macroporous copolymer is obtained.
(2).With the existence of inert medium, the above described macroporous copolymer matrix reacted with the Lewis acid as the catalyst at 50-120°C for 5-20hours though the alkylate reaction between the aromatic nucleus. Then recycle the inert organic medium to obtain the product. The dosage of inert organic medium is 4-8 times of the quantity of the copolymer matrix , as to the dosage of Lewis acid is about 5-30%.

3. The process of claim 2 is that monomer mixture constitutes of monovinyl aromatic monomer, polyvinyl aromatic monome used as crosslinking agent and the polar aliphatic unsaturated monomer, as amount of the polar aliphatic unsaturated monomer is 0-10%wt , 50-80%wt of polyvinyl aromatic monomer , and others are monovinyl aromatic monomer.

4. The process of claim 3 is that monovinyl aromatic monomer includes, for example, styrene, alkyl-substituted styrene, vinylnaphthalene or other similar unsaturated monomer, and as to the alkyl-substituted styrene, there are also several selections as following: methyl styrene, ethylstyrene, diethylstyrene, methylethylstyrene or dimethylstyrene and so on. The most optimized choice of monovinyl aromatic monomer is styrene or alkyl-substituted styrene.

5. The process of claim 3 is that polyvinyl aromatic monomer used as the crosslinking agent, such as divinylbenzene, trivinylbenzene, divinyltoluene, divinylnaphthalene,divinylxylene or divinylethylbenzene and so on, the most optimized is divinylbenzene.

6. The process of claim 3 is that polar aliphatic unsaturated monomer can be selected among the followings: acrylic acid, methacrylic acid, low alkyl ester of acrylic acid or low alkyl ester of methacrylic acid, and the low alkyl ester of acrylic acid can be selected among methyl acrylate, ethyl acrylate, propyl acrylate, isopropyl acrylate or acrylonitrile; as to the low alkyl ester of methacrylic acid can be selected among the followings: ethyleneglycol di(meth)acrylate, trimethylolpropane tri(meth)acrylate, ethyl methacrylate, propyl methacrylate, isopropyl methacrylate , and the most optimized is low alkyl ester of methacrylic acid or acrylonitrile.

7. The process of claim 2 is that the porogen agent which is used for preparing the ground substance of crosslinked macroporous copolymer can be selected one or more among the followings: toluene, xylene, C₄-C₁₀ alkanol, C₆-C₁₂ saturated hydrocarbon, alkyl-substituted cyclohexane; C₄-C₁₀ alkanol can be selected among n-butanol, tertiary amyl alcohol, or diethyl hexanol ; C₆-C₁₂ saturated hydrocarbon can be selected among heptane, isooctane or gasoline, and other alkyl-substituted cyclohexane; The amount of the optimized porogen agent is 100-300%wt of the total monomers mixture.

8. The process of claim 2 is that the initiator of polymerization in procedure (1) can be selected among the followings: benzoperoxide, lauroyl peroxide or AZO compound, such as azobisisobutyronitrile, etc., and the amount of initiator is 0.5-2% wt of the total monomers.

9. The process of claim 2 is that the inertia organic medium described in procedure (2) is selected among dichloroethane, dichloropropane, nitrobenzene, chlorobenzene or dichlorobenzene.

10. The process of claim 2 is that the Lewis acid catalyst is selected among anhydrous aluminium trichloride, anhydrous ferric chloride, anhydrous zinc chloride, stannic chloride or boron trifluoride and so on.
